# EUROPÄISCHE PATENTANMELDUNG

(11) **EP 4 108 306 A1**
(43) Veröffentlichungstag der Anmeldung: **28.12.2022**
(21) Anmeldenummer: 21181624.4
(22) Anmeldetag: 25.06.2021
(51) Int. Cl.: B01D 3/00, B01D 3/40, C07C 7/08

(54) **EXTRAKTIONSDESTILLATIONSKOLONNENSYSTEM UND DESSEN EINSATZ BEI DER TRENNUNG VON BUTENEN AUS C4-KOHLENWASSERSTOFFSTRÖMEN**

(71) Anmelder: Evonik Operations GmbH, 45128 Essen (DE)
(72) Erfinder: LUTZE, Philip, 46535 Dinslaken (DE); PEITZ, Stephan, 45739 Oer-Erkenschwick (DE); RIX, Armin Matthias, 45770 Marl (DE); SIX, Tanita Valèrie, 44309 Dortmund (DE); SCHRÖDER, Moritz, 48153 Münster (DE); PAUL, Niklas, 45770 Marl (DE)
(74) Vertreter: Evonik Patent Association

(57) **Zusammenfassung**

Gegenstand der Erfindung ist ein Extraktionsdestillationskolonnensystem, welches eine Kombinationskolonne (1) und eine Seitenverstärkerkolonne aufweist. Weiterhin betrifft die vorliegende Erfindung ein Verfahren zur Abtrennung von Butenen aus C4-Kohlenwasserstoffströmen unter Verwendung des Extraktionsdestillationskolonnensystems.

## Beschreibung

Die vorliegende Erfindung betrifft ein Extraktionsdestillationskolonnensystem, welches eine Kombinationskolonne (1) und eine Seitenverstärkerkolonne (2) aufweist. Weiterhin betrifft die vorliegende Erfindung ein Verfahren zur Abtrennung von Butenen aus C4-Kohlenwasserstoffströmen unter Verwendung des Extraktionsdestillationskolonnensystems.

Die Abtrennung von Butenen aus C4-Kohlenwasserstoffströmen, die neben den Butenen auch Butane enthalten, ist ein bekanntes Verfahren. Dazu wird üblicherweise die Extraktivdestillation eingesetzt, wobei ein aprotisches Lösemittel (z. B. N-Methyl-2-pyrrolidon (NMP) oder Acetonitril (ACN)) verwendet wird, um die relative Flüchtigkeit der Alkane gegenüber den Alkenen zu erhöhen. Anlagen zur Durchführung einer entsprechenden Extraktivdestillation umfassen üblicherweise zwei getrennte Kolonnen. In der einen Kolonne, dem Absorber, werden die Butene im Lösemittel gelöst und die Butane als Kopfprodukt abgetrennt. Das mit den Butenen beladene Lösemittel wird anschließend in einer Stripkolonne, dem Desorber, bei erhöhter Temperatur und/oder reduziertem Druck von den Butenen befreit, die am Kopf des Desorbers in angereicherter Form anfallen. Das von den Butenen befreite Lösemittel wird dann zum Absorber zurückgefahren.

Bei den bekannten Anlagen mit (räumlich) getrennten Absorber- und Desorberkolonnen wird ein flüssiger Strom vom Absorber zum Desorber geführt. Dies geschieht üblicherweise mit einer Pumpe. Eine solche Pumpe ist teuer in der Anschaffung und führt letztlich auch zu signifikanten Betriebs- und Instandhaltungskosten. Weiterhin brauchen entsprechende Anlagen ausreichend Platz für die jeweiligen Kolonnen und sonstigen Aufbauten. Ausreichend Platz ist jedoch nicht immer vorhanden. Es besteht daher Bedarf Anlagen zur Abtrennung von Butenen aus C4-Kohlenwasserstoffströmen kosten- und platzsparender zu bauen bzw. zu betreiben.

Die Aufgabe der vorliegenden Erfindung war demzufolge die Bereitstellung eines Extraktiosdestillationskolonnensystems, das weniger Platz benötigt und günstiger zu betreiben ist. Weiterhin war die Aufgabe ein platz- und kostensparendes Verfahren zur Abtrennung von Butenen bereitzustellen.

Diese Aufgaben können durch die in Anspruch 1 vorgeschlagene Ausführung des Extraktionsdestillationskolonnensystems und das in Anspruch 7 beschriebene Verfahren gelöst werden. Bevorzugte Ausführungsformen sind in den Unteransprüchen angegeben.

Das erfindungsgemäße Extraktionsdestillationskolonnensystem ist ein Extraktionsdestillationskolonnensystem zur Abtrennung von Butenen aus einem C4-Kohlenwasserstoffstrom, der zumindest Butene und Butane enthält, unter Verwendung eines Lösemittels, wobei das Extraktionsdestillationskolonnensystem eine Kombinationskolonne (1) und eine Seitenverstärkerkolonne (2) aufweist,
wobei die Kombinationskolonne (1), von oben nach unten betrachtet, zumindest die folgenden unterschiedlichen Abschnitte aufweist: einen Kopfabschnitt, in dem ein im Vergleich zum eingesetzten C4-Kohlenwasserstoffstrom an Butanen angereicherter Strom anfällt; einen Füllkörperabschnitt, der mindestens zwei Füllkörperbetten umfasst; einen Sammelabschnitt, der mindestens zwei Flüssigkeitssammler, vorzugsweise Kaminböden, umfasst; einen Regenerierabschnitt, der mindestens ein Füllkörperbett umfasst; und einen Sumpfabschnitt, in dem das Lösemittel anfällt; und wobei an der Kombinationskolonne (1) mindestens zwei Seitenverdampfersysteme (5, 6) angeordnet sind, in denen jeweils eine flüssige Phase aus einem der Flüssigkeitssammler des Sammelabschnitts zumindest teilweise verdampft und anschließend über jeweils einen Einlass (5a, 6a) zurück in den Sammelabschnitt geleitet wird, wobei der Einlass (5a) räumlich betrachtet mindestens einen Flüssigkeitssammler oberhalb des Einlasses (6a) angeordnet ist;
wobei die Seitenverstärkerkolonne (2) zumindest zwei Trennböden oder mindestens ein Füllkörper- oder Packungsbett aufweist und mit einer gasförmigen Phase gespeist wird, die unterhalb des Einlasses (5a), aber oberhalb oder auf gleicher Höhe des Einlasses (6a) aus dem Sammelabschnitt der Kombinationskolonne (1) anfällt. In einer bevorzugten Ausführungsform der vorliegenden Erfindung weist der Kopfabschnitt Böden oder Füllkörper auf.

Es sei darauf hingewiesen, dass die jeweilige flüssige Phase, die aus einem der Flüssigkeitssammler des Sammelabschnitts entnommen und durch die Seitenverdampfersysteme (5, 6) geleitet wird, überwiegend in flüssiger Form vorliegt. Da in der Kombinationskolonne aber auch gasförmige Phasen vorhanden sind, kann es sein, dass Teile der gasförmigen Phase mit der flüssigen Phase, beispielsweise in Form von Gasbläschen, mitgerissen werden, wenn auch in (sehr) geringem Umfang. Die Formulierung flüssige Phase soll dies explizit nicht ausschließen.

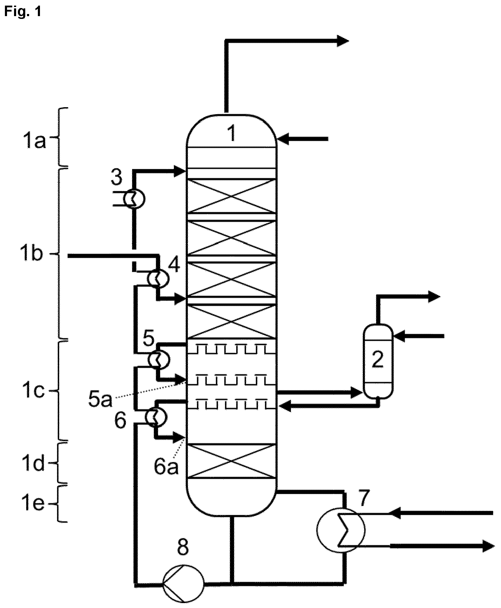

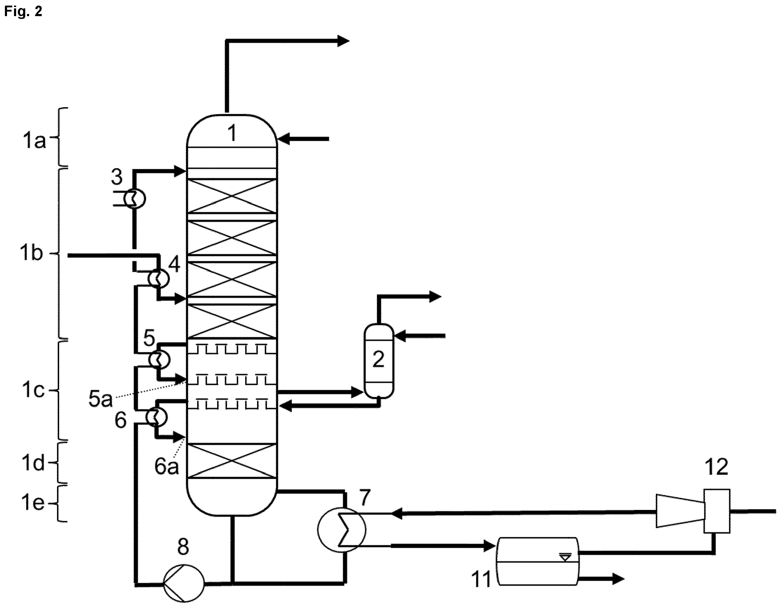

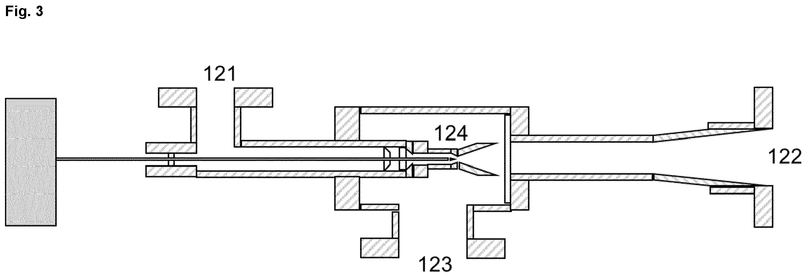

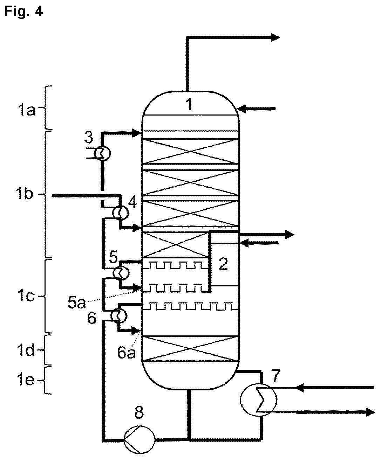

Die vorliegende Kombinationskolonne (1) mit den betreffenden Abschnitten stellt erfindungsgemäße eine Kombination aus einem Absorber und einem Teil des Desorbers von bekannten Extraktionsdestillationskolonnen dar. Der, räumlich betrachtet, obere Teil der Kombinationskolonne, d.h. der Kopfabschnitt, der Füllkörperabschnitt und ein Teil des Sammelabschnitts, sind mit dem Absorber, bei dem die Butene aus dem C4-Kohlenwasserstoffstrom in das Lösemittel übergehen, vergleichbar. Der, räumlich betrachtet, untere Teil der Kombinationskolonne, d.h. ein Teil des Sammelabschnitts, der Regenerierabschnitt und der Sumpfabschnitt, sind mit einem Teil des Desorbers (räumlich betrachtet der untere Teil eines Desorbers), bei dem die Butene aus dem Lösemittel entfernt werden, vergleichbar. Die Seitenverstärkerkolonne (2) ist mit dem, räumlich betrachtet, oberen Teil eines Desorbers vergleichbar, da dort die aus dem Lösemittel abgetrennten Butene am Kopf anfallen. Die Seitenverstärkerkolonne (2) kann als eigene Kolonne ausgestaltet sein (vgl. Fig. 1 und 2) oder in die Kombinationskolonne (1) integriert werden (vgl. Fig. 3 und 4), d. h. mit der Kombinationskolonne (1) in einem gemeinsamen Kolonnenmantel vorliegen.

Die Vorteile des erfindungsgemäße Extraktionsdestillationskolonnensystems sind offensichtlich. Zwischen der Kombinationskolonne (1) und der Seitenverstärkerkolonne (2) wird in beiden Ausführungsformen keine Pumpe benötigt. Die für Anschaffung und Betrieb anfallenden Kosten fallen weg. Weiterhin kann eine erfindungsgemäße Seitenverstärkerkolonne (2) sehr viel kleiner ausfallen als die zweite Kolonne (Desorber) von bekannten Anlagen. Das erfindungsgemäße Kolonnensystem benötigt somit auch weniger Platz. Wird die Seitenverstärkerkolonne (1) in die Kombinationskolonne (1) integriert, wird noch weniger Platz benötigt. Zudem sind keine Rohrleitungen zur Seitenverstärkerkolonne (2) notwendig.

Die vorliegende Erfindung betrifft die Abtrennung von Butenen aus C4-Kohlenwasserstoffströmen mittels dem erfindungsgemäßen Extraktionsdestillationskolonnensystem. C4-Kohlenwasserstoffströme enthalten üblicherweise neben den Butenen auch Alkane (n-Butan, Isobutan). Der Begriff Butane wird im Rahmen der vorliegenden Erfindung dabei so verstanden, dass er - sofern nichts anderes bezeichnet ist - sowohl n-Butan als auch Isobutan meint. Im erfindungsgemäßen Verfahren können daher alle C4-Kohlenwasserstoffströme eingesetzt werden, die zumindest Butene und Butane enthalten, sofern die Mengen, in denen die Butene und/oder Butane vorhanden sind, eine wirtschaftliche Durchführung des Verfahrens zulassen. In einer bevorzugten Ausführungsform der vorliegenden Erfindung besteht der eingesetzte C4-Kohlenwasserstoffstrom im Wesentlichen, d.h. zu mehr als 98 Gew.-%, vorzugsweise zu mehr als 99 Gew.-% aus Butanen und Butenen. Die entsprechenden Ströme können in geringen Mengen auch Verunreinigungen oder andere Kohlenwasserstoffe, wie 1,3-Butadien, C3- oder C5-Kohlenwasserstoffe, enthalten.

Als Lösemittel wird ein flüssiges Lösemittel eingesetzt, in dem sich vorrangig die Butene des eingesetzten, gasförmigen C4-Kohlenwasserstoffstroms lösen. Geeignete Lösemittel sind aprotische Lösemittel, beispielsweise N-Methyl-2-pyrrolidon (NMP). Vorzugsweise wird NMP als Lösemittel eingesetzt. In einer weiterhin bevorzugten Ausführungsform der vorliegenden Erfindung enthält das Lösemittel Wasser, insbesondere im Bereich von 1 bis 10 Gew.-%, vorzugsweise von 4 bis 9 Gew.-%, jeweils bezogen auf die Gesamtmenge an Lösemittel. Die Kombinationskolonne (1) des erfindungsgemäßen Extraktionsdestillationskolonnensystems weist insbesondere einen geeigneten Einlass für das Lösemittel auf, der vorzugsweise oberhalb des ersten Füllkörperbetts des Füllkörperabschnitts oder oberhalb des zweiten Füllkörperbetts des Füllkörperabschnitts angeordnet ist.

Die Kombinationskolonne (1) des erfindungsgemäßen Extraktionsdestillationskolonnensystems weist insbesondere auch einen geeigneten Einlass für den C4-Kohlenwasserstoffstrom auf, aus dem die Butene abgetrennt werden sollen. Das flüssige Lösemittel wird räumlich betrachtet oberhalb des Einlasses für den C4-Kohlenwasserstoffstroms zur Kombinationskolonne (1) gegeben, d. h. der Einlass für das Lösemittel befindet sich oberhalb des Einlasses für den C4-Kohlenwasserstoffstrom. In einer bevorzugten Ausführungsform ist der Einlass für den C4-Kohlenwasserstoffstrom, von oben gesehen, mindestens 1 Füllkörperbett unterhalb des Einlasses für das Lösemittel angeordnet. Das flüssige Lösemittel wird in der Kombinationskolonne nach unten rieseln und mit dem (aufsteigenden) dampfförmigen C4-Kohlenwasserstoffstrom in Kontakt gebracht, wodurch ein Teil des C4-Kohlenwasserstoffstroms, der überwiegend Butene enthält, in das Lösemittel übergeht.

Bevor der C4-Kohlenwasserstoffstrom über den Einlass in die Kombinationskolonne (1) gelangt, wird der Strom vorzugsweise zumindest teilweise verdampft. Dazu kann das Extraktionskolonnensystem einen Feedverdampfer (4) aufweisen, durch den der C4-Kohlenwasserstoffstrom vor dem Einlass in die Kombinationskolonne (1) zumindest teilweise verdampft wird. Vorteilhaft ist, dass die Verdampfung nicht vollständig in der Kombinationskolonne (1) erfolgen muss.

Das erfindungsgemäße Extraktionsdestillationskolonnensystem weist zwei Seitenverdampfersysteme (5, 6) auf. Die beiden Seitenverdampfersysteme (5, 6) sind untereinander angeordnet, wobei die dort zumindest teilweise verdampften Ströme über je einen Einlass (5a, 6a) zurück in den Sammelabschnitt der Kombinationskolonne (1) geführt werden. Einlass (5a) gehört dabei zum Seitenverdampfersystem (5) und Einlass (6a) zum Seitenverdampfersystem (6). Das erfindungsgemäßes Seitenverdampfersystem (5) weist vorzugsweise einen einzigen oder zwei parallel oder sequenziell angeordnete Verdampfer auf. Sofern das Seitenverdampfersystem (5) zwei parallel oder sequenziell angeordnete Verdampfer aufweist, kann ein zusätzlicher Einlass in den Sammelabschnitt der Kombinationskolonne (1) vorhanden sein. Dieser zusätzliche Einlass würde sich, räumlich betrachtet oberhalb oder auf gleicher Höhe des Einlasses (5a) befinden. Das erfindungsgemäßes Seitenverdampfersystem (6) weist vorzugsweise einen einzigen oder zwei parallel oder sequenziell angeordnete Verdampfer auf. Sofern das Seitenverdampfersystem (6) zwei parallel oder sequenziell angeordnete Verdampfer aufweist, kann ein zusätzlicher Einlass in den Sammelabschnitt der Kombinationskolonne (1) vorhanden sein. Dieser zusätzliche Einlass würde sich, räumlich betrachtet, unterhalb oder auf gleicher Höhe des Einlasses (6a) befinden.

Sollten das Seitenverdampfersystem (5) und/oder das Seitenverdampfersystem (6) zwei parallel oder sequenziell angeordnete Verdampfer aufweisen, kann es notwendig sein, dass der der Sammelabschnitt der Kombinationskolonne (1) mehr als zwei Flüssigkeitssammler aufweist, auf die die zurückgeführten Ströme aus dem jeweiligen Verdampfer gegeben und/oder von denen sie abgezogen werden.

Am Sumpfabschnitt der Kombinationskolonne (1) des Extraktionsdestillationskolonnensystems ist vorzugsweise ein Sumpfverdampfer (7) angeordnet, durch den die im Sumpf anfallende Flüssigkeit zumindest teilweise verdampft wird, um noch Reste von Butenen aus dem Lösemittel auszugasen. Im Sumpf fällt dann regeneriertes, d. h. an den Butenen abgereichertes, heißes Lösemittel an. Das im Sumpf anfallende Lösemittel wird vorzugsweise zum Einlass für das Lösemittel zurückgeleitet. In einer bevorzugten Ausführungsform wird die Wärme des zum Einlass der Kombinationskolonne (1) zurückgeführte Lösemittel zur Wärmeintegration verwendet, d. h. die Wärme des Lösemittels wird vorzugsweise zur Verdampfung im Feedverdampfer (4) und zur Verdampfung in den Seitenverdampfersystemen (5, 6) eingesetzt. Feedverdampfer (4) und Seitenverdampfersysteme (5, 6) sind vorzugsweise mit einem Wärmetauscher ausgestattet, die diese Wärmeübertragung ermöglicht.

Durch die Wärmeintegration wird dem Lösemittel Wärme entzogen. Der Grund dafür ist nicht nur, dass damit andere Ströme oder Kolonnen geheizt werden sollen, sondern in erster Linie die Abkühlung des Lösemittels. Wird dem Lösemittel bei der Wärmeintegration ausreichend Wärme entzogen, weist also eine geeignete Temperatur auf, kann das Lösemittel direkt in den Füllkörperabschnitt der Kombinationskolonne (1) gefahren werden. Es ist jedoch auch denkbar, dass das Lösemittel trotz der vorliegenden Wärmeintegration noch nicht die richtige Temperatur aufweist. In so einem Fall kann das Lösemittel nach der Wärmeintegration und vor dem Eintritt in die Kombinationskolonne (1) durch einen Restkühler geleitet werden, um auf eine geeignete Temperatur gekühlt zu werden.

Wärme ist eine Prozessgröße. Die zu- oder abgeführte Wärme entspricht der Änderung der Inneren Energie abzüglich der verrichteten Arbeit. Bei den in dieser Erfindung verwendeten Begriffe Wärme, Wärmetransport und Wärmeintegration wird diese Definition stets zu Grunde gelegt.

Die Ausgestaltung der Seitenverstärkerkolonne (2) ist durch zumindest zwei Trennböden oder durch ein Füllkörper- oder Packungsbett gekennzeichnet. Die Seitenverstärkerkolonne wird mit einer gasförmigen Phase gespeist, die unterhalb des Einlasses (5a), aber oberhalb oder auf gleicher Höhe des Einlasses (6a) aus dem Sammelabschnitt der Kombinationskolonne (1) abgenommen wird. Die gasförmige Phase umfasst insbesondere überwiegend Butene, kann jedoch auch u. a. Reste an Lösemitteln enthalten, beispielsweise in Form mitgerissener Tröpfchen. Es ist erfindungsgemäße bevorzugt, dass die Lösemittelreste abgetrennt werden und zurück zur Kombinationskolonne geführt werden, beispielsweise durch eine geeignete Leitung. Der Einlass für die zurückgeführten Lösemittelreste befindet demgemäß sich im Sammelabschnitt der Kombinationskolonne (1). Am Kopf der Seitenverstärkerkolonne (2) kann dann ein im Vergleich zum eingesetzten C4-Kohlenwasserstoffstrom an Butenen angereichter Strom entnommen werden.

Ein weiterer Gegenstand der vorliegenden Erfindung ist ein Verfahren zur Abtrennung von Butenen aus C4-Kohlenwasserstoffströmen durch Extraktivdestillation unter Verwendung des erfindungsgemäßen und vorgenannt beschriebenen Extraktionsdestillationskolonnensystems. Die Abtrennung der Butene erfolgt dabei zumindest durch die folgenden Schritte:
Zuführen eines zumindest teilweise in einem Feedverdampfer (4) verdampften C4-Kohlenwasserstoffstroms in den Füllkörperabschnitt der Kombinationskolonne (1) und Zuführen des flüssigen Lösemittels mindestens ein Füllkörperbett oberhalb des C4-Kohlenwasserstoffstroms, wodurch der C4-Kohlenwasserstoffstrom und das Lösemittel miteinander in Kontakt gebracht werden und überwiegend Butene aus dem C4-Kohlenwasserstoffstrom in das Lösemittel unter Erhalt eines beladenen Lösemittels übergehen;
wobei das beladene Lösemittel in einem Flüssigkeitssammler des Sammelabschnitts gesammelt, durch ein erstes Seitenverdampfersystem (5) gefahren, dort zumindest teilweise verdampft und dann über den Einlass (5a) zurück in den Sammelabschnitt geführt wird;
wobei eine in einem weiteren Flüssigkeitssammler des Sammelabschnitts anfallende flüssige Phase durch das zweite Seitenverdampfersystem (6) gefahren wird, dort zumindest teilweise verdampft und dann über den Einlass (6a) zurück in den Sammelabschnitt geführt wird, von wo aus die flüssige Phase in den Regenerierabschnitt gelangt;
wobei die unter dem letzten Füllkörperbett des Regenerierabschnitts anfallende flüssige Phase, welche das Lösemittel und Reste an Butenen und/oder Butanen enthält, durch einen Sumpfverdampfer (7) gefahren und dann in den Sumpfabschnitt geleitet wird, wodurch noch im Lösemittel vorhandene Butene und/oder Butane zumindest teilweise ausgegast werden, das so erhaltene Lösemittel als Sumpfstrom abgezogen und zum Füllkörperabschnitt zurückgeführt wird; und
Bereitstellen eines gasförmigen Stroms, der zumindest Butene und Reste an Lösemittel enthält, unterhalb des Einlasses (5a), aber oberhalb oder auf gleicher Höhe des Einlasses (6a) aus dem Sammelabschnitt und Zuführen dieses gasförmigen Stroms zur Seitenverstärkerkolonne (2), wodurch am Kopf der Seitenverstärkerkolonne ein an Butenen angereicherter Strom anfällt, dadurch gekennzeichnet, dass die Wärme des als Sumpfstrom abgenommen Lösemittels zumindest teilweise zur Wärmeintegration verwendet wird, in dem Wärme des Lösemittels im ersten Seitenverdampfersystem (5), im zweiten Seitenverdampfersystem (6) und im Feedverdampfer (4) übertragen wird.

Das vorliegende Verfahren betrifft die Abtrennung von Butenen aus butenhaltigen C4-Kohlenwasserstoffströmen. Diese Ströme enthalten üblicherweise neben den Butenen auch Alkane (n-Butan, Isobutan). Der Begriff Butane wird im Rahmen der vorliegenden Erfindung dabei so verstanden, dass er - sofern nichts anderes bezeichnet ist - sowohl n-Butan als auch Isobutan meint. Im erfindungsgemäßen Verfahren können daher alle C4-Kohlenwasserstoffströme eingesetzt werden, die zumindest Butene und Butane enthalten, sofern die Mengen, in denen die Butene und/oder Butane vorhanden sind, eine wirtschaftliche Durchführung des Verfahrens zulassen. In einer bevorzugten Ausführungsform der vorliegenden Erfindung besteht der eingesetzte C4-Kohlenwasserstoffstrom im Wesentlichen, d.h. zu mehr als 98 Gew.-%, vorzugsweise zu mehr als 99 Gew.-% aus Butanen und Butenen. Die entsprechenden Ströme können auch Verunreinigungen oder andere Kohlenwasserstoffe, wie 1,3-Butadien oder C5-Kohlenwasserstoffe, in geringen Mengen enthalten.

Als Lösemittel wird ein flüssiges Lösemittel eingesetzt, in dem sich vorrangig die Butene des eingesetzten, gasförmigen C4-Kohlenwasserstoffstroms lösen. Geeignete Lösemittel sind aprotische Lösemittel, beispielsweise N-Methyl-2-pyrrolidon (NMP). Vorzugsweise wird NMP als Lösemittel eingesetzt. In einer weiterhin bevorzugten Ausführungsform der vorliegenden Erfindung enthält das Lösemittel Wasser, insbesondere im Bereich von 1 bis 10 Gew.-%, vorzugsweise von 4 bis 9 Gew.-%, jeweils bezogen auf die Gesamtmenge an Lösemittel.

Das flüssige Lösemittel wird räumlich betrachtet oberhalb des Einlasses für den C4-Kohlenwasserstoffstroms zur Kombinationskolonne (1) gegeben. Das flüssige Lösemittel wird in der Kombinationskolonne (1) nach unten rieseln und mit dem (aufsteigenden) dampfförmigen C4-Kohlenwasserstoffstrom in Kontakt gebracht, wodurch ein Teil des C4-Kohlenwasserstoffstroms, der überwiegend Butene enthält, in das Lösemittel übergeht, wodurch ein beladenes Lösemittel entsteht. Der C4-Kohlenwasserstoffstrom und das Lösemittel werden insbesondere im Gegenstrom miteinander in Kontakt gebracht. In einer bevorzugten Ausführungsform der vorliegenden Erfindung umfasst der Teil des C4-Kohlenwasserstoffstroms, der in das Lösemittel übergeht, mindestens 70 Gew.-%, besonders bevorzugt mindestens 80 Gew.-% Butene, bezogen auf die Zusammensetzung des in das Lösemittel übergegangenen Teils des C4-Kohlenwasserstoffstroms. Daraus ergibt sich insbesondere, dass mindestens 80%, besonders bevorzugt mindestens 90% der im eingesetzten C4-Kohlenwasserstoffstrom enthaltenen Butene in das Lösemittel übergehen. Das beladene Lösemittel läuft in der Kombinationskolonne (1) nach unten und wird in einem geeigneten Flüssigkeitssammler des Sammelabschnitts, insbesondere einem Kaminboden, gesammelt. Vom Flüssigkeitssammler wird das beladene Lösemittel abgenommen, durch das erste Seitenverdampfersystem (5) gefahren und dann unterhalb des Flüssigkeitssammlers über den Einlass (5a) in den Sammelabschnitt zurückgeführt wird. Das erfindungsgemäße Seitenverdampfersystem (5) umfasst vorzugsweise oder besteht vorzugsweise aus einem Once-Through-Verdampfer, bei dem das beladene Lösemittel nur einmal durch den Verdampfer geleitet wird. Dadurch lassen sich möglichst niedrige Temperaturen realisieren, wodurch Fouling verhindert werden kann. Das erfindungsgemäßes Seitenverdampfersystem (5) weist weiterhin bevorzugt einen einzigen oder zwei parallel oder sequenziell angeordnete Verdampfer auf. Sofern das Seitenverdampfersystem (5) zwei parallel oder sequenziell angeordnete Verdampfer aufweist, kann ein zusätzlicher Einlass in den Sammelabschnitt der Kombinationskolonne (1) vorhanden sein. Dieser zusätzliche Einlass würde sich, räumlich betrachtet oberhalb oder auf gleicher Höhe des Einlasses (5a) befinden.

In einem weiteren Flüssigkeitssammler des Sammelabschnitts fällt eine flüssige Phase an, die dort abgenommen, durch das zweite Seitenverdampfersystem (6) gefahren und dann unterhalb dieses Flüssigkeitssammlers über den Einlass (6a) in den Sammelabschnitt zurückgeführt wird. Von dort gelangt die verbliebene (nicht verdampfte) flüssige Phase in den Regenerierabschnitt. Das erfindungsgemäße Seitenverdampfersystem (6) umfasst vorzugsweise oder besteht vorzugsweise aus einem Once-Through-Verdampfer, bei dem die flüssige Phase nur einmal durch den Verdampfer geleitet wird. Dadurch lassen sich möglichst niedrige Temperaturen realisieren, wodurch Fouling verhindert werden kann. Das erfindungsgemäße Seitenverdampfersystem (6) weist weiterhin bevorzugt einen einzigen oder zwei parallel oder sequenziell angeordnete Verdampfer auf. Sofern das Seitenverdampfersystem (6) zwei parallel oder sequenziell angeordnete Verdampfer aufweist, kann ein zusätzlicher Einlass in den Sammelabschnitt der Kombinationskolonne (1) vorhanden sein. Dieser zusätzliche Einlass würde sich, räumlich betrachtet, unterhalb oder auf gleicher Höhe des Einlasses (6a) befinden.

Die Flüssigkeitssammler, aus denen das beladene Lösemittel respektive die flüssige Phase abgenommen und zu den Seitenverdampfersystemen (5, 6) geführt wird, sind, räumlich betrachtet, dementsprechend untereinander angeordnet, wobei der Flüssigkeitssammler, über den das beladene Lösemittel zum Seitenverdampfersystem (5), gelangt oberhalb des Flüssigkeitssammlers, über den die flüssige Phase zum Seitenverdampfersystem (6) gelangt, angeordnet ist. Die in den Seitenverdampfersystemen (5, 6) zumindest teilweise verdampften Ströme werden über je einen Einlass (5a, 6a) zurück in den Sammelabschnitt der Kombinationskolonne (1) geführt. Der gasförmige Teil dieser jeweils zurückgeführten Ströme wird nach oben aufsteigen, während der flüssige Teil dieser Ströme in der Kombinationskolonne weiter nach unten gelangt.

Die flüssige Phase läuft vom Sammelabschnitt über den Regenerierabschnitt, der mindestens ein Füllkörperbett umfasst, weiter nach unten. Unter dem letzten Füllkörperbett des Regenerierabschnitts wird die flüssige Phase vorzugsweise in einem geeigneten Sammler gesammelt. Die unter dem letzten Füllkörperbett ankommende oder optional im Sammler gesammelte flüssige Phase, welche das Lösemittel und Reste an Butenen und/oder Butanen enthält, durch einen Sumpfverdampfer (7) gefahren und dann in den Sumpfabschnitt geleitet wird, wodurch noch im Lösemittel vorhandene Butene und/oder Butane zumindest teilweise ausgegast werden. Im Sumpf der Kombinationskolonne (1) liegt erhöhte Temperatur vor. Die Temperatur im Sumpf der Kombinationskolonne (1) beträgt vorzugsweise zwischen 120 und 200 °C, weiterhin bevorzugt zwischen 130 und 195 °C.

Der Sumpfverdampfer (7) ist vorzugsweise ein Once-Through-Verdampfer, bei dem die flüssige Phase nur einmal durch den Verdampfer geleitet wird. Dadurch lassen sich möglichst niedrige Temperaturen realisieren, wodurch Fouling verhindert werden kann. Weiterhin werden kleinere mittlere Temperaturdifferenzen ermöglicht, wodurch die Wärmeübertragung vereinfacht werden kann. Der Sumpfverdampfer (7) kann auch mehrstufig ausgestaltet sein, d. h. es können mehrere Wärmetauscher bzw. mehrere Verdampfer vorhanden sein, die zum Sumpfverdampfer (7) gehören. Im Sumpfabschnitt der Kombinationskolonne fällt dann das Lösemittel an, welches zum Füllkörperabschnitt der Kombinationskolonne (1) zurückgeführt wird, insbesondere zum entsprechenden Einlass.

Ein wichtiges Merkmal der vorliegenden Erfindung ist die Wärmeintegration unter Verwendung der Wärme des Lösemittels auf dem Weg vom Sumpf zum Füllkörperabschnitt der Kombinationskolonne (1) und ggf. des Heißkondensats, welches im Sumpfverdampfer (7) anfällt. Die Wärme des im Sumpf der Kombinationskolonne (1) abgenommen Lösemittels, vorzugsweise des NMPs, wird erfindungsgemäß zur Wärmeintegration verwendet, in dem Wärme des Lösemittels im ersten Seitenverdampfersystem (5), im zweiten Seitenverdampfersystem (6) und im Feedverdampfer (4) übertragen wird.

Die Wärme zur Verdampfung Sumpfverdampfer (7) kann in einem Wärmeübertrager durch Wärmeübertragung von einem geeigneten Wärmeträgermedium eingetragen werden. Das Wärmeträgermedium kann insbesondere Heizdampf sein, der als Mittel- oder Hochdruckdampf eingesetzt wird. Als Heizdampf ist ein Mitteldruckdampf bevorzugt, welcher eine Temperatur von 150 bis 270 °C, vorzugsweise von 160 bis 250 °C aufweist. Der Mitteldruckdampf weist vorzugsweise einen Druck von 15 bis 30 bar absolut, besonders bevorzugt von 17 bis 25 bar absolut auf. Als Heizdampf kann auch ein Dampf mit einem Druck von > 30 bar absolut eingesetzt werden. Ein solcher Heizdampf kann auch als Hochdruckdampf bezeichnet werden.

Der zur Verdampfung im Sumpfverdampfer (7) eingesetzte Heizdampf kann im Wärmetauscher zumindest teilweise kondensieren, wodurch ein Heißkondensat bei einem Druck von 10 bis 20 bar absolut, vorzugsweise 12 bis 17 bar absolut und einer Temperatur von 150 bis 210 °C, vorzugsweise 160 bis 200 °C anfällt. Nach dem Wärmetauscher ist vorzugsweise ein Kondensatbehälter angeordnet, in dem das Heißkondensat vom Dampf getrennt werden kann. In dem Kondensatbehälter liegt vorzugsweise ein geringerer Druck vor als im Wärmetauscher auf der Heizdampfseite. Aufgrund des geringeren Drucks kann ein Teil des Heißkondensats verdampfen, wodurch der gesamte Dampf, d. h. der nicht kondensierte Anteil des Heizdampfes und das im Kondensatbehälter durch Druckentspannung verdampfte Heißkondensat, im Kondensatbehälter als Niederdruckdampf anfällt. Niederdruckdampf weist vorliegend vorzugsweise einen Druck von mehr als 0 bar und weniger als 10 bar absolut auf. Die Temperatur des Niederdruckdampfs beträgt vorzugsweise 100 bis 180 °C.

Der dort anfallende Niederdruckdampf enthält noch Energie, die ausgenutzt werden kann. Aus energetischer und wirtschaftlicher Sicht ist das jedoch nicht sinnvoll. Diese Energie kann jedoch in einer bevorzugten Ausgestaltung der vorliegenden Erfindung genutzt werden. Dazu kann der zur Verdampfung Sumpfverdampfer (7) eingesetzte Heizdampf mittels eines, vorzugsweise regelbaren Dampfstrahlers (Thermokompressors) zur Verfügung gestellt werden. Der Thermokompressor wird dann sowohl mit dem eingesetzten Heizdampf, der beispielsweise aus einem entsprechenden Dampfnetz stammt, hier insbesondere der bevorzugt eingesetzte Mitteldruckdampf, als auch mit dem Niederdruckdampf aus dem Kondensatbehälter gespeist, wodurch ein Mischdampf entsteht, der dementsprechend das Wärmeträgermedium für den Sumpfverdampfer (7) ist. Der Mischdampf ist in dieser Ausgestaltung demnach der Heizdampf. Ein solcher Dampfstrahler ist so ausgestaltet, dass er mit einem Treibdampf betrieben wird und durch einen Unterdruck (Staudruck im Dampfstrahler) Saugdampf aus einem Behälter ansaugen kann, wodurch dann der Mischdampf entsteht, der als Wärmeträgermedium eingesetzt wird. Der Treibdampf ist im vorliegenden Fall der Heizdampf bzw. der Mitteldruckdampf, mit dem der Niederdruckdampf als Saugdampf aus dem Kondensatbehälter angesaugt und mit dem Treibdampf vermischt wird.

Der Vorteil einer solchen Ausgestaltung ist offensichtlich. Die Energie des im Kondensatbehälter anfallenden Niederdruckdampfes kann genutzt und damit Energie und Kosten gespart werden. Eine solche Verfahrensweise kann auch aus einem anderen Grund vorteilhaft sein. Der eingesetzte Dampfstrahler kann dahingehend regelbar sein, dass die Mengen von Mittel- bzw. Hochdruck- und Niederdruckdampf, beispielsweise in Abhängigkeit von bestimmten Prozessparametern, eingestellt werden können. Die Saugdampfmenge stellt sich dabei über die Treibdampfmenge ein. Die Mengen von Mitteldruck- und Niederdruckdampf können beispielsweise in Abhängigkeit von der Temperatur in der Kombinationskolonne (1) eingestellt werden.

Am Kopf der Kombinationskolonne (1) fällt dann insbesondere ein im Vergleich zum eingesetzten C4-Kohlenwasserstoffstrom an Butanen angereicherter Strom an. Der Kopfdruck in der Kombinationskolonne (1) kann dabei zwischen 1 und 7 bar absolut, vorzugsweise zwischen 2 und 6,5 bar absolut betragen. Der an Butanen angereicherte Strom kann zusätzlich Wasser enthalten, welches aus dem Lösemittel stammt. Dieses Wasser kann in einem nachfolgenden Schritt abgetrennt werden. Dabei wird der an Butanen angereicherte Strom am Kopf der Kombinationskolonne entnommen und einer ein- oder mehrstufigen Kondensation unterworfen, wobei ein wasserhaltiger Strom und ein butanhaltiger Produktstrom auskondensiert werden. Diese beiden Ströme können in einer geeigneten Vorrichtung, beispielsweise einem Euter, voneinander getrennt werden. Der vom butanhaltigen Produktstrom abgetrennte wasserhaltige Strom kann je nach seiner Zusammensetzung zur Kombinationskolonne (1) oder zur Seitenverstärkerkolonne (2) geführt und/oder teilweise aus dem Verfahren ausgeschleust werden.

Der so aus der Kondensation erhaltene butanhaltige Produktstrom kann noch geringe Mengen an Wasser enthalten, insbesondere in einer Menge von bis zu 1500 Gew.-ppm, bezogen auf die Gesamtzusammensetzung des butanhaltigen Produktstroms. Außerdem kann der aus der Kondensation erhaltene butanhaltige Produktstrom noch Restbutene enthalten, wobei die Ströme üblicherweise weniger als 20 Gew.-%, vorzugsweise weniger als 15 Gew.-%, besonders bevorzugt weniger als 5 Gew.-% an Butenen, bezogen auf die Gesamtzusammensetzung des butanhaltigen Produktstroms, enthalten.

Je nach den Anforderungen an den erhaltenen butanhaltigen Produktstrom kann es erforderlich sein, dass der butanhaltige Produktstrom nach der Kondensation einer Trocknung, vorzugsweise in einer Trocknungskolonne, unterworfen wird, um das noch enthaltene Wasser abzutrennen. Vorzugsweise weist der butanhaltige Produktstrom nach der Trocknung eine maximale Menge an Wasser von 50 Gew.-ppm, vorzugsweise von 25 Gew.-ppm auf. Das bei der Trocknung anfallende Wasser bzw. der Brüdenstrom der Trocknung kann zur Kondensation an der Kombinationskolonne (1) zurückgeführt werden.

Unterhalb des Einlasses (5a), aber oberhalb oder auf gleicher Höhe des Einlasses (6a) fällt am Sammelabschnitt der Kombinationskolonne (1) ein gasförmiger Strom an und wird der Seitenverstärkerkolonne zugeführt. Die gasförmige Phase enthält überwiegend Butene, kann aber u. a. auch noch Reste an Butanen und Lösemittel, beispielsweise in Form von Tröpfchen, enthalten. Diese Seitenverstärkerkolonne (2) weist, wie bereits erwähnt, zumindest zwei Trennböden oder mindestens ein Füllkörper- oder Packungsbett auf, die insbesondere der Abtrennung von Lösemittel dienen. Der Kopfdruck in der Seitenverstärkerkolonne kann zwischen 1 und 7 bar absolut, vorzugsweise zwischen 2 und 6,5 bar absolut betragen. Der Druck von Kombinationskolonne (1) und Seitenverstärkerkolonne (2) ist vorzugsweise gekoppelt.

Am Kopf der Seitenverstärkerkolonne (2) fällt dann insbesondere ein im Vergleich zu dem eingesetzten C4-Kohlenwasserstoffstrom an Butenen angereicherte Strom an. Dieser an Butenen angereicherte Strom kann zusätzlich Wasser enthalten, welches aus dem Lösemittel stammt. Dieses Wasser kann in einem nachfolgenden Schritt abgetrennt werden. Dabei wird der an Butenen angereichte Strom am Kopf der Seitenverstärkerkolonne (2) entnommen und einer ein- oder mehrstufigen Kondensation unterworfen, wobei ein wasserhaltiger Strom, der neben Wasser auch noch Reste an Organik enthalten kann, und ein butenhaltiger Produktstrom auskondensiert werden. Diese beiden Ströme können in einer geeigneten Vorrichtung, beispielsweise einem Euter, voneinander getrennt werden. Der vom butenhaltigen Produktstrom abgetrennte wasserhaltiger Strom kann anschließend zurück zur Seitenverstärkerkolonne (2) oder zur Kombinationskolonne geführt werden. Möglich ist auch eine Ausschleusung des ganzen oder eines Teils des wasserhaltigen Stroms, um die darin enthaltene Organik zurückzuhalten.

In einer bevorzugten Ausführungsform der vorliegenden Erfindung ist die Kondensation des am Kopf der Seitenverstärkerkolonne (2) abgenommenen an Butenen angereicherten Stroms zweistufig ausgestaltet, wobei in einer ersten Stufe ein wasserhaltiger Strom auskondensiert wird, das dann zur Seitenverstärkerkolonne (2) zurückgeführt wird, und in der zweiten Stufe der butenhaltige Produktstrom auskondensiert wird. Es kann jedoch auch sein, dass in der zweiten Stufe auch noch vorhandenes Wasser auskondensiert. Dieses restliche Wasser kann über eine geeignete Vorrichtung, beispielsweise einen Euter, vom butenhaltigen Produktstrom abgetrennt werden.

Der aus der Kondensation erhaltene butenhaltige Produktstrom enthält vorzugsweise weniger als 20 Gew.-%, weiterhin bevorzugt weniger als 16 Gew.-% an Butanen bezogen auf die Gesamtzusammensetzung des butenhaltigen Produktstroms. Der aus der Kondensation erhaltene butenhaltige Produktstrom weist stattdessen vorzugsweise einen Butengehalt von mindestens 70 Gew.-%, weiterhin bevorzugt von mindestens 75 Gew.-%, besonders bevorzugt von mindestens 86 Gew.-% bezogen auf die Gesamtzusammensetzung des butenhaltigen Produktstroms auf.

Im unteren Teil der Seitenverstärkerkolonne (2) bzw. im Sumpf der Seitenverstärkerkolonne (2) kann ein flüssiger Sumpfstrom anfallen, der im Wesentlichen aus der Kombinationskolonne (1) eingetragenes Lösemittel enthält. Dieser Strom fällt nur in der Ausführungsform an, wo die Seitenverstärkerkolonne (2) als eigene Kolonne ausgestaltet ist (vgl. Fig. 1 und 2). Dieser flüssige Sumpfstrom kann aus der Seitenverstärkerkolonne (2) zur Kombinationskolonne (1) (zurück)geleitet werden. Der zurückgeführte Strom an einer geeigneten Stelle auf die Kombinationskolonne (1) gegeben, beispielsweise dort, wo es aus konzentrationstechnischer Sicht von Vorteil ist.

Die vorliegende Erfindung wird nachfolgend anhand von Abbildungen erläutert. Die Abbildungen dienen der Veranschaulichung, sind aber nicht einschränkend zu verstehen.
Fig. 1 zeigt die grundsätzliche Ausgestaltung der vorliegenden Erfindung. Der flüssige C4-Kohlenwasserstoffstrom wird über einen Feedverdampfer (4) verdampft und in den Füllkörperabschnitt (1b) der Kombinationskolonne (1) geleitet. Das Lösemittel wird über einen Restkühler (3) - wenn notwendig - auf die gewünschte Temperatur gebracht und ebenfalls in Füllkörperabschnitt (1b) der Kombinationskolonne (1) geleitet, wobei der Einlass räumlich gesehen oberhalb des Einlasses für den C4-Kohlenwasserstoffstrom ist, im vorliegenden Fall oberhalb des ersten Füllkörperbettes. Am Kopfabschnitt (1a) der Kombinationskolonne (1) fällt der an Butanen angereicherte Strom an, der abgenommen wird. Eine mögliche Kondensation ist hier nicht gezeigt, lediglich die Rückführung eines möglichen Teilstroms ist durch den Pfeil angedeutet. Im Sammelabschnitt (1c) der Kombinationskolonne (1) wird das beladene Lösemittel in einem Flüssigkeitssammler gesammelt, was in der Abbildung durch den Kaminboden angedeutet ist. Dort wird zumindest ein Teil des beladenen Lösemittels abgenommen und über das erste Seitenverdampfersystem (5) und anschließend über den Einlass (5a) zurück in den Sammelabschnitt (1c) geführt. Eine flüssige Phase wird in einem weiteren Flüssigkeitssammler gesammelt, von dort abgenommen und über das zweite Seitenverdampfersystem (6) über den Einlass (6a) zurück in den Sammelabschnitt geführt, von wo aus die flüssige Phase in den Regenerierabschnitt (1d) gelangt. Unter dem letzten Füllkörperbett wird die dort anfallende flüssige Phase abgenommen und über den Sumpfverdampfer (7) zum Sumpf (1e) der Kombinationskolonne (1) geführt. Aus dem Sumpf (1e) der Kombinationskolonne (1) wird dann das an Butenen abgereicherte Lösemittel abgenommen und mittels einer Pumpe (8) über die Seitenverstärkersysteme (5, 6) und den Feedverdampfer zum Füllkörperabschnitt der Kombinationskolonne (1) zurückgeführt. Zwischen dem Einlass (5a) und dem Einlass (6a) wird ein gasförmiger Strom abgenommen und zur Seitenverstärkerkolonne (2) geleitet, wo restliches Lösemittel und Wasser von den Butenen abgetrennt werden sollen. Am Kopf der Seitenverstärkerkolonne (2) fällt der an Butenen angereicherte Strom an. Dieser Strom kann einer ein- oder mehrstufigen Kondensation unterworfen werden, was in der Abbildung nicht gezeigt ist. Lediglich ein möglicher Rückführstrom ist durch den Pfeil angedeutet. Im Sumpf der Seitenverstärkerkolonne (2) fällt hier eine flüssige Phase an, die zurück zum Sammelabschnitt der Kombinationskolonne (1) geführt werden kann.
Fig. 2 zeigt eine weiterhin bevorzugte Ausführungsform der vorliegenden Erfindung, bei der am Sumpfverdampfer (7) ein Dampfstrahler (12) vorhanden ist. Dieser Dampfstrahler wird mit dem regulären Heizdampf, also beispielsweise dem Mitteldruckdampf aus dem Dampfnetz, und dem Niederdruckdampf, der im Kondensatbehälter (11) anfällt, gespeist, wodurch ein Mischdampf entsteht, der dann als Heizdampf zum Sumpfverdampfer (7) verwendet wird. Die Funktionsweise eines Dampfstrahlers wird bei der Beschreibung von Fig. 3 erläutert. Alles weitere entspricht den vorherigen Ausführungen zu Fig. 1.
Fig. 3 zeigt den schematischen Aufbau eines Dampfstrahlers (12). Der Treibdampf (121) ist hier der Heizdampf, insbesondere der Mitteldruckdampf aus dem Dampfnetz. Der Saugdampf (123) ist der Niederdruckdampf aus dem Kondensatbehälter. Beide werden über die Regeleinheit (124) vermischt und über den Ausgang als Mischdampf (122) zum Sumpfverdampfer (7) geführt. Über die Regeleinheit können die Mengen von Treibdampf und Saugdampf eingestellt werden, wodurch Druck und Temperatur des Mischdampfes und damit die mögliche Heizleistung beeinflusst werden können.
Fig. 4 zeigt einen weiteren Gegenstand der vorliegenden Erfindung. Die Funktionsweise entspricht dem in Fig. 1 dargestellten Verfahren und unterscheidet sich lediglich in der Bauform. Demzufolge sind die Kombinationskolonne (1) und die Seitenverstärkerkolonne (2) zwar unterschiedliche Einheiten mit den erfindungsgemäß beschriebenen Funktionen. Die Seitenverstärkerkolonne ist dabei aber räumlich in die Kombinationskolonne integriert worden, d. h. baulich in einem einzigen Kolonnenmantel ausgeführt. Die Seitenverstärkerkolonne (2) ist räumlich gesehen nach unten offen ausgestaltet und wird durch eine aufsteigende gasförmige Phase, die zumindest Butene und Reste an Lösemittel und Wasser enthält. An der Seitenverstärkerkolonne (2) fällt der an Butenen angereicherte Strom an. Dieser Strom kann einer ein- oder mehrstufigen Kondensation unterworfen werden, was in der Abbildung nicht gezeigt ist. Lediglich ein möglicher Rückführstrom ist durch den Pfeil angedeutet.

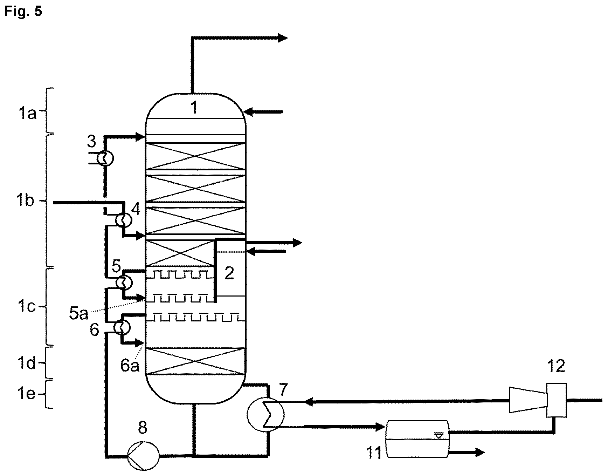

Fig. 5 zeigt eine weiterhin bevorzugte Ausführungsform der vorliegenden Erfindung, bei der am Sumpfverdampfer (7) ein Dampfstrahler (12) vorhanden ist. Dieser Dampfstrahler wird mit dem regulären Heizdampf, also beispielsweise dem Mitteldruckdampf aus dem Dampfnetz, und dem Niederdruckdampf, der im Kondensatbehälter (11) anfällt, gespeist, wodurch ein Mischdampf entsteht, der dann als Heizdampf zum Sumpfverdampfer (7) verwendet wird. Die Funktionsweise eines Dampfstrahlers wird bei der Beschreibung von Fig. 3 erläutert. Alles weitere entspricht den vorherigen Ausführungen zu Fig. 1.

## Patentansprüche

1. Extraktionsdestillationskolonnensystem zur Abtrennung von Butenen aus einem C4-Kohlenwasserstoffstrom, der zumindest Butene und Butane enthält, unter Verwendung eines Lösemittels, wobei das Extraktionsdestillationskolonnensystem eine Kombinationskolonne (1) und eine Seitenverstärkerkolonne (2) aufweist,
wobei die Kombinationskolonne (1), von oben nach unten betrachtet, zumindest die folgenden unterschiedlichen Abschnitte aufweist: einen Kopfabschnitt, in dem ein im Vergleich zum eingesetzten C4-Kohlenwasserstoffstrom an Butanen angereicherter Strom anfällt; einen Füllkörperabschnitt, der mindestens zwei Füllkörperbetten umfasst; einen Sammelabschnitt, der mindestens zwei Flüssigkeitssammler, vorzugsweise Kaminböden, umfasst; einen Regenerierabschnitt, der mindestens ein Füllkörperbett umfasst; und einen Sumpfabschnitt, in dem das Lösemittel anfällt; und wobei an der Kombinationskolonne (1) mindestens zwei Seitenverdampfersysteme (5, 6) angeordnet sind, in denen jeweils eine flüssige Phase aus einem der Flüssigkeitssammler des Sammelabschnitts zumindest teilweise verdampft und anschließend über jeweils einen Einlass (5a, 6a) zurück in den Sammelabschnitt geleitet wird, wobei der Einlass (5a) räumlich betrachtet mindestens einen Flüssigkeitssammler oberhalb des Einlasses (6a) angeordnet ist;
wobei die Seitenverstärkerkolonne (2) zumindest zwei Trennböden oder mindestens ein Füllkörper- oder Packungsbett aufweist und mit einer gasförmigen Phase gespeist wird, die unterhalb des Einlasses (5a), aber oberhalb oder auf gleicher Höhe des Einlasses (6a) aus dem Sammelabschnitt der Kombinationskolonne (1) anfällt.

2. Extraktionsdestillationskolonnensystem nach Anspruch 1, wobei das Extraktionskolonnensystem einen Feedverdampfer (4) aufweist, durch den der C4-Kohlenwasserstoffstrom zumindest teilweise verdampft wird, bevor er in die Kombinationskolonne (1) geführt wird.

3. Extraktionsdestillationskolonnensystem nach Anspruch 2, wobei das im Sumpf der Kombinationskolonne (1) anfallende Lösemittel zum Einlass für das Lösemittel zurückgeleitet wird.

4. Extraktionsdestillationskolonnensystem nach Anspruch 3, wobei das Lösemittel dabei zur Wärmeintegration verwendet wird, in dem Wärme des Lösemittels im ersten Seitenverdampfersystem (5), im zweiten Seitenverdampfersystem (6) und im Feedverdampfer (4) übertragen wird.

5. Extraktionsdestillationskolonnensystem nach einem der vorhergehenden Ansprüche, wobei am Sumpfabschnitt der Kombinationskolonne (1) ein Sumpfverdampfer (7) angeordnet ist, durch den die im Sumpf anfallende Flüssigkeit zumindest teilweise verdampft wird, um noch Reste von Butenen aus dem Lösemittel auszugasen.

6. Extraktionsdestillationskolonnensystem nach einem der vorherigen Ansprüche, wobei am Kopf der Seitenverstärkerkolonne (2) ein an Butenen angereichter Strom entnommen wird.

7. Verfahren zur Abtrennung von Butenen aus einem C4-Kohlenwasserstoffstrom, der zumindest Butene und Butane enthält, durch Extraktivdestillation in einem Extraktionsdestillationskolonnensystem nach einem der Ansprüche 1 bis 6, wobei die Abtrennung der Butene durch folgende Schritte erfolgt:
Zuführen eines zumindest teilweise in einem Feedverdampfer (4) verdampften C4-Kohlenwasserstoffstroms in den Füllkörperabschnitt der Kombinationskolonne (1) und Zuführen des flüssigen Lösemittels mindestens ein Füllkörperbett oberhalb des C4-Kohlenwasserstoffstroms, wodurch der C4-Kohlenwasserstoffstrom und das Lösemittel miteinander in Kontakt gebracht werden und überwiegend Butene aus dem C4-Kohlenwasserstoffstrom in das Lösemittel unter Erhalt eines beladenen Lösemittels übergehen;
wobei das beladene Lösemittel in einem Flüssigkeitssammler des Sammelabschnitts gesammelt, durch ein erstes Seitenverdampfersystem (5) gefahren, dort zumindest teilweise verdampft und dann über den Einlass (5a) zurück in den Sammelabschnitt geführt wird;
wobei eine in einem weiteren Flüssigkeitssammler des Sammelabschnitts anfallende flüssige Phase durch ein zweites Seitenverdampfersystem (6) gefahren wird, dort zumindest teilweise verdampft und dann über den Einlass (6a) zurück in den Sammelabschnitt geführt wird, von wo aus die flüssige Phase in den Regenerierabschnitt gelangt;
wobei die unter dem letzten Füllkörperbett des Regenerierabschnitts anfallende flüssige Phase, welche das Lösemittel und Reste an Butenen und/oder Butanen enthält, durch einen Sumpfverdampfer (7) gefahren und dann in den Sumpfabschnitt geleitet wird, wodurch noch im Lösemittel vorhandene Butene und/oder Butane zumindest teilweise ausgegast werden, das so erhaltene Lösemittel als Sumpfstrom abgezogen und zum Füllkörperabschnitt zurückgeführt wird; und
Bereitstellen eines gasförmigen Stroms, der zumindest Butene und Reste an Lösemittel enthält, unterhalb des Einlasses (5a), aber oberhalb oder auf gleicher Höhe des Einlasses (6a) aus dem Sammelabschnitt und Zuführen dieses gasförmigen Stroms zur Seitenverstärkerkolonne (2), wodurch am Kopf der Seitenverstärkerkolonne ein an Butenen angereicherter Strom anfällt, **dadurch gekennzeichnet, dass**
die Wärme des als Sumpfstrom abgenommen Lösemittels zumindest teilweise zur Wärmeintegration verwendet wird, in dem Wärme des Lösemittels im ersten Seitenverdampfersystem (5), im zweiten Seitenverdampfersystem (6) und im Feedverdampfer (4) übertragen wird.

8. Verfahren nach Anspruch 7, wobei das eingesetzte Lösemittel NMP ist.

9. Verfahren nach Anspruch 7 oder 8, wobei die Wärme zur Verdampfung im Sumpfverdampfer (7) in einem Wärmetauscher durch Wärmeübertragung mit einem geeigneten Wärmeträgermedium, insbesondere Heizdampf, eingetragen wird.

10. Verfahren nach Anspruch 9, wobei der eingesetzte Heizdampf im Wärmetauscher zumindest teilweise kondensiert und dadurch ein Heißkondensat bei einem Druck von 10 bis 20 bar absolut, vorzugsweise 12 bis 17 bar absolut und einer Temperatur von 150 bis 210 °C, vorzugsweise 160 bis 200 °C anfällt, das zu einem Kondensatbehälter geleitet wird.

11. Verfahren nach Anspruch 10, wobei im Kondensatbehälter ein geringerer Druck vorliegt als im Wärmetauscher auf der Heizdampfseite und dadurch ein Teil des Heißkondensats wieder verdampft und als Niederdruckdampf anfällt.

12. Verfahren nach Anspruch 11, wobei der Heizdampf für den Sumpfverdampfer (7) mittels eines Dampfstrahlers, der mit Hochdruck- oder Mitteldruckdampf und dem im Kondensatbehälter anfallenden Niederdruckdampf gespeist wird, zur Verfügung gestellt wird.

13. Verfahren nach einem der vorhergehenden Ansprüche, wobei die Seitenverstärkerkolonne (2) einen Sumpf aufweist, in dem ein flüssiger Sumpfstrom anfällt, der aus diesem Sumpf zur Kombinationskolonne (1) geleitet wird.

14. Verfahren nach einem der vorhergehenden Ansprüche, wobei der Sumpfverdampfer (7) ein Once-Through-Verdampfer ist.

15. Verfahren nach einem der vorhergehenden Ansprüche, wobei mindestens eines der oder beiden Seitenverdampfersysteme (5, 6) einen Once-Through-Verdampfer umfasst oder daraus besteht.
